# EUROPEAN PATENT SPECIFICATION

(11) **EP 1 996 923 B1**
(45) Date of publication and mention of the grant of the patent: **27.06.2012**
(21) Application number: 07752007.0
(22) Date of filing: 02.03.2007
(51) Int. Cl.: G01N 24/00, G01N 33/68

(54) **METHODS FOR DISTINGUISHING ISOMERS USING MASS SPECTROMETRY**
VERFAHREN ZUR UNTERSCHEIDUNG VON ISOMEREN MITHILFE DER MASSENSPEKTROMETRIE
PROCÉDÉS PERMETTANT DE DISTINGUER DES ISOMÈRES PAR SPECTROMÉTRIE DE MASSE

(30) Priority: 02.03.2006 US 778483 P
(43) Date of publication of application: 03.12.2008
(73) Proprietor: PerkinElmer Health Sciences, Inc., Boston MA 02118 (US)
(72) Inventor: CERDA, Blas, Milford, Massachusetts 01757 (US)
(74) Representative: Williams, Gareth Owen
(86) International application number: PCT/US2007/005279
(87) International publication number: WO 2007/103124

(56) References cited:
- WO-A-2004/095032
- WO-A-2006/128419
- MARTENS-LOBENHOFFER J ET AL: "Determination of arginine and asymmetric dimethylarginine (ADMA) in human plasma by liquid chromatography/mass spectrometry with the isotope dilution technique" JOURNAL OF MASS SPECTROMETRY, WILEY, CHICHESTER, GB, vol. 39, no. 11, 7 October 2004 (2004-10-07), pages 1287-1294, XP002399826 ISSN: 1076-5174
- JENS MARTENS-LOBENHOFFER ET AL: "Measurement of asymmetric dimethylarginine (ADMA) in human plasma: from liquid chromatography estimation to liquid chromatography-mass spectrometry quantification" EUROPEAN JOURNAL OF CLINICAL PHARMACOLOGY, SPRINGER, BERLIN, DE, vol. 62, no. 1, 11 October 2005 (2005-10-11), pages 61-68, XP019512882 ISSN: 1432-1041
- BÖGER ET AL: "Asymmetric dimethylarginine (ADMA): A novel risk factor for endothelial Dysfunction" CIRCULATION, LIPPINCOTT WILLIAMS & WILKINS, US, vol. 98, no. 18, 3 November 1998 (1998-11-03), pages 1842-1847, XP000909286 ISSN: 0009-7322
- MARTENS-LOBENHOFFER J. ET AL.: 'Fast and Efficient Determination of Arginine, Symmetric Dimethylarginine, and Asymmetric Dimethylarginine in Biological Fluids by Hydrophilic-Interaction Liquid Chromatography-Electrospray Tandem Mass Spectrometry' CLIN. CHEM. vol. 52, no. 3, March 2006, pages 488 - 493, XP001247520
- SCHWEDHELM E.: 'Quantification of ADMA: analytical approaches' VASCULAR MEDICINE vol. 10, 2005, pages S89 - S95, XP009072520
- SCHWEDHELM ET AL.: 'Liquid Chromatography-Tandem Mass Spectrometry Method for the Analysis of Asymmetric Dimethylarginine in Human Plasma' CLIN. CHEM. vol. 51, no. 7, 2005, pages 1268 - 1271, XP008130301

## Description

### SUMMARY

This invention relates, *inter alia*, to distinguishing molecular isomers using mass spectrometry. For example, this disclosure features a method of detecting one or both isomers of dimethylarginine, using tandem mass spectrometry.

Martens - Lobenhoffer et al, Clin Chem Vol 52 No 3 2006, pages 488-493 discloses a method of distinguishing dimethylarginine isomers by ionising a sample to generate ions, selecting ions having a mass to charge ratio, fragmenting selected ions, and detecting one or both of ADMA and SDMA by detecting daughter ions. This publication also discloses a method of diagnosing a disorder characterised by altered ADMA or SDMA levels by measuring the levels of dimethylarginine isomers by mass spectrometry. This publication discloses the use of chromatographic separation prior to ionisation.

The present invention provides a method of distinguishing dimethylarginine isomers, the method being in accordance with independent claim 1. A further aspect of the injection relates to a method of diagnosing a disorder characterised by altered ADMA or SDMA levels, in accordance with independent claim 17, or to a method of evaluating a subject's response to a therapeutic agent, the method being in accordance with claim 22. A further aspect of the invention provides a kit for detecting ADMA and SDMA, as recited in claim 23. Further aspects of the invention are described in the dependent claims.

These methods have a variety of applications and can be used to detect dimethylarginine in a sample (e.g., a sample from a synthesis or a sample from a subject). In the context of diagnostics, the methods can be used to obtain a metabolic profile for a subject (e.g., a human). A metabolic profile is information that includes information about the status of one or both members of a pair of isomers of a metabolite, e.g., one or both of asymmetric dimethylarginine (ADMA) symmetric dimethylarginine (SDMA), the two isomers of dimethylarginine: 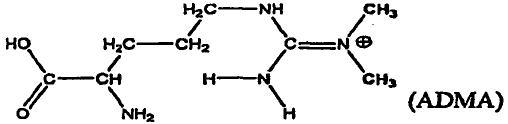 and 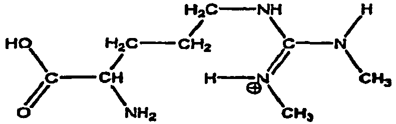 (SDMA).
In some embodiments, a metabolic profile includes information about other molecules, e.g., other metabolites. The resultant metabolic profile can be used for assessing the health state of a subject (e.g., a human), such as presence or absence of a disorder, e.g., a vascular disorder (e.g., a cardiovascular disorder or hypertensive disorder).

In one aspect, the disclosure features a method of distinguishing dimethylarginine isomers. The method includes the steps of: ionizing a sample to generate ions; selecting ions having a mass-to-charge ratio (*m*/*z*) in an *m*/*z* range;fragmenting the selected ions to produce daughter ions; and detecting one or both of asymmetric dimethylarginine (ADMA) and symmetric dimethylarginine (SDMA) in the sample by detecting one or both of a daughter ion *m*/*z* signal at a *m*/*z* unique to ADMA and a daughter ion *m*/*z* signal at a *m*/*z* unique to SDMA.

In another aspect, the disclosure features a method of distinguishing dimethylarginine isomers, which method includes the steps of: ionizing a sample to generate ions; selecting ions having a mass-to-charge ratio (*mlz*) in an *m*/*z* range;
fragmenting the selected ions to produce daughter ions; and detecting one or both of the presence of a daughter ion *m*/*z* signal at about *m*/*z* 46 and the presence of a daughter ion *m*/*z* signal at about *m*/*z* 172, wherein a daughter ion *m*/*z* signal at about *m*/*z* 46 indicates that the sample comprises asymmetric dimethylarginine (ADMA) and a daughter ion *m*/*z* signal at about *m*/*z* 172 indicates that the sample comprises symmetric dimethylarginine (SDMA). The *m*/*z* range can be about *m*/*z* 203.

In another aspect, the disclosure features a method of detecting symmetric dimethylarginine (SDMA), which method includes the steps of: ionizing a sample to generate ions; selecting ions having a mass-to-charge ratio (*m*/*z*) in an *m*/*z* range; fragmenting the selected ions to produce daughter ions; and detecting symmetric dimethylarginine (SDMA) in the sample by detecting a daughter ion *m*/*z* signal at about *m*/*z* 172. The *m*/*z* range can be about *m*/*z* 203

In another aspect, the disclosure features a method of asymmetric dimethylarginine (ADMA). The method includes the steps of: ionizing a sample to generate ions; selecting ions having a mass-to-charge ratio (*m*/*z*) in an *m*/*z* range; fragmenting the selected ions to produce daughter ions; and detecting asymmetric dimethylarginine (ADMA) in the sample by detecting a daughter ion *m*/*z* signal at about *m*/*z* 46. The *m*/*z* range can be about *m*/*z* 203.

In some embodiments of any of the methods described above, an internal standard is added to the sample prior to ionizing the sample. The internal standard can be, or contain, a dimethylarginine isomer that includes at least one heavy atom. The internal standard can contain or be a reference SDMA isomer having at least one heavy atom, and a reference ADMA isomer having at least one heavy atom and wherein the references SDMA and ADMA isomers have different atomic weights from one another.

In another aspect, the disclosure features a method of detecting dimethylarginine isomers, which method includes the steps of: providing a sample that includes one or more internal standards, wherein at least one internal standard is a dimethylarginine isomer containing at least one heavy atom; ionizing a sample to generate ions; selecting ions having.an *m*/*z* range corresponding to the range being suitable to isolate dimethylarginine isomers (e.g., naturally occurring) and selecting ions in one or more additional *m*/*z* ranges, the ranges being suitable to isolate the one or more internal standards; fragmenting the selected ions to produce daughter ions; detecting asymmetric dimethylarginine (ADMA) and/or symmetric dimethylarginine (SDMA) in the sample by detecting one or more of a daughter ion *m*/*z* signal unique to ADMA and/or one or more of a daughter ion *m*/*z* signal unique to SDMA using the daughter ions; and detecting the one or more internal standards.

In some embodiments, the *m*/*z* range corresponds to the range being suitable to isolate dimethylarginine isomers is about *m*/*z* 203. In some embodiments, the sample can includes a first internal standard that is an SDMA isomer having at least one heavy atom, and a second internal standard that is an ADMA isomer having at least one heavy atom and wherein the SDMA and ADMA isomers have different atomic weights. In some embodiments, the sample includes a first internal standard that is an SDMA isomer having at least one heavy atom, and a second internal standard that is an ADMA isomer having at least one heavy atom and wherein the SDMA and ADMA isomers have the same atomic weights.

In some embodiments of any of the methods described above, the method can further include the step of measuring the level of SDMA and/or ADMA in the sample.

In some embodiments of any of the methods described above, the sample that is ionized can be a biological sample. The biological sample can be, or contain, blood, serum, plasma, lymph, amnionic fluid, saliva, cerebral-spinal fluid, lacrimal fluid, mucus, urine, sputum, or sweat.

In some embodiments of any of the methods described above, the sample that is ionized can contains compounds in addition to dimethylarginine. The sample that is ionized can contain compounds that do not co-migrate on a size exclusion chromatography column.

In some embodiments of any of the methods described above, one or more additional analytes can be detected. The one or more additional analytes can be metabolic biomolecules. The one or more additional analytes can be amino acids or acylcarnitines. The one or more additional analytes are selected from the group consisting of: citrulline, dimethylamine, arginine, orthnithine, homocysteine, and creatine. In some embodiments, the one or more additional analytes can be measured, e.g., using tandem mass spectrometry.

In some embodiments of any of the methods described above, the sample is not chemically modified prior to ionizing the sample. In some embodiments of any of the methods described above, the sample is chemically modified prior to ionizing the sample.

In some embodiments of any of the methods described above, the level of a signal at about *m*/*z* 46 and the level of a signal at about *m*/*z* 172 can be measured, e.g., by determining the result of a function depending on the daughter ion *m*/*z* signal at about *m*/*z* 46 and the daughter ion *m*/*z* signal at about *m*/*z* 172.

In another aspect, the disclosure features a method of diagnosing a disorder characterized by altered ADMA or SDMA levels, the method comprising: providing a biological sample from a subject; ionizing the biological sample to generate ions; selecting ions having a mass-to-charge ratio *(m*/*z)* in an *m*/*z* range; fragmenting the selected ions to produce daughter ions; and measuring the level of ADMA as a function of a daughter ion *m*/*z* signal at about *m*/*z* 46 and the level of SDMA as a function of a daughter ion *m*/*z* signal at about *m*/*z* 172, wherein an altered level of one or both of ADMA and SDMA in the biological sample relative to the level in a reference sample is an indication that the subject has, or is at risk of developing, a disorder characterized by altered ADMA or SDMA levels. The disorder can be characterized by increased ADMA levels and/or increased SDMA levels. The disorder characterized by altered ADMA or SDMA levels can be a vascular disorder and/or a hypertensive disorder. The hypertensive disorder can be preeclampsia. The m/z range can be about m/z 203. The biological sample that is ionized can contain compounds in addition to dimethylarginine. The subject can be a mammal, e.g., a human. The vascular disorder can be a cardiovascular disorder or a hypertensive disorder. The hypertensive disorder can be preeclampsia. The cardiovascular disorder can be atherosclerosis. The vascular disorder can be diabetes, hypercholesterolemia, renal insufficiency, or hypertension. In some embodiments, the level of a daughter ion m/z signal at about m/z 46 and the level of a daughter ion m/z signal at about m/z 172 can be measured. The reference sample can be from subject not having, not suspected of having, or not at risk of developing a cardiovascular disorder. The biological sample can be, or contain, blood, serum, plasma, lymph, amnionic fluid, saliva, cerebral-spinal fluid, lacrimal fluid, mucus, urine, sputum, or sweat.

In some embodiments, one or more additional analytes can be measured. The one or more additional analytes can be citrulline, dimethylamine, arginine, orthnithine, homocysteine, and creatine. The one or more additional analytes can be amino acids or acylcarnitines. The one or more additional analytes can be measured, e.g., using mass spectrometry.

In some embodiments, an internal standard can be added to the biological sample prior to ionizing the sample. internal standard can be, or contain, a dimethylarginine isomer that includes at least one heavy atom. The internal standard can be, or contain, an SDMA isomer having at least one heavy atom, and an ADMA isomer having at least one heavy atom and wherein the SDMA and ADMA isomers have different atomic weights.

In yet another aspect, the disclosure features a method of diagnosing a vascular disorder in a subject, which method includes the steps of: providing a biological sample from a subject; ionizing the biological sample to generate ions; selecting ions having a mass-to-charge ratio (*m*/*z*) in an *m*/*z* range; fragmenting the selected ions to produce daughter ions; and measuring one or both of the level of ADMA as a function of a daughter ion *m*/*z* signal at about *m*/*z* 46 and the level of SDMA as a function of a daughter ion *m*/*z* signal at about *m*/*z* 172, wherein an elevated level of one or both of ADMA and SDMA in the biological sample relative the level in a reference sample is an indication that the subject has, or is at risk of developing, a vascular disorder. The m/z range can be about m/z 203. The biological sample that is ionized can contain compounds in addition to dimethylarginine. The subject can be a mammal, e.g., a human. The vascular disorder can be a cardiovascular disorder or a hypertensive disorder. The hypertensive disorder can be preeclampsia. The cardiovascular disorder can be atherosclerosis. The vascular disorder can be diabetes, hypercholesterolemia, renal insufficiency, or hypertension. In some embodiments, the level of a daughter ion m/z signal at about *mlz* 46 and the level of a daughter ion *m*/*z* signal at about *m*/*z* 172 can be measured. The reference sample can be from subject not having, not suspected of having, or not at risk of developing a cardiovascular disorder.

In some embodiments, one or more additional analytes can be measured. The one or more additional analytes can be citrulline, dimethylamine, arginine, orthnithine, homocysteine, and creatine. The one or more additional analytes can be amino acids or acylcarnitines. The one or more additional analytes can be measured, e.g., using mass spectrometry. The biological sample can be, or contain, blood, serum, plasma, lymph, amnionic fluid, saliva, cerebral-spinal fluid, lacrimal fluid, mucus, urine, sputum, or sweat. The biological sample that is ionized can be, or contain, compounds in addition to dimethylarginine.

In some embodiments, an internal standard can be added to the biological sample prior to ionizing the sample. internal standard can be, or contain, a dimethylarginine isomer that includes at least one heavy atom. The internal standard can be, or contain, an SDMA isomer having at least one heavy atom, and an ADMA isomer having at least one heavy atom and wherein the SDMA and ADMA isomers have different atomic weights.

In another aspect, the disclosure provides a method of diagnosing preeclampsia in a subject, which method includes the steps of: providing a biological sample from a subject; ionizing the biological sample to generate ions; selecting ions having a mass-to-charge ratio (*m*/*z*) in an *m*/*z* range; fragmenting the selected ions to produce daughter ions; and measuring one or both of the level of ADMA as a function of a daughter ion *m*/*z* signal at about *m*/*z* 46 and the level of SDMA as a function of a daughter ion *m*/*z* signal at about *m*/*z* 172, wherein an elevated level of one or both of ADMA and SDMA in the biological sample relative the level in a reference sample is an indication that the subject has, or is at risk of developing, preeclampsia. The *m*/*z* range can be about *m*/*z* 203. The subject can be a mammal, e.g., a human.

In yet another aspect, the disclosure provides a method of evaluating a subject's response to a therapeutic agent. The method can include the steps of:
providing a biological sample from a subject treated with a therapeutic agent; ionizing the biological sample to generate ions; selecting ions having a mass-to-charge ratio (*m*/*z*) in an *m*/*z* range; fragmenting the selected ions to produce daughter ions; and measuring one or both of the level of ADMA as a function of a daughter ion *m*/*z* signal at about *m*/*z* 46 and the level of SDMA as a function of a daughter ion *m*/*z* signal at about *m*/*z* 172, wherein the same level or an elevated level of one or both of ADMA and SDMA in the biological sample relative the level in a biological sample obtained from the subject prior to treatment is an indication that the subject is not responding to the treatment and a reduced level of one or both of ADMA and SDMA in the biological sample relative to the biological sample obtained from the subject prior to treatment is an indication that the subject is responding to the treatment. The m/z range can be about m/z 203. The subject can be a mammal, e.g., a human. In some embodiments, one or more additional analytes can be measured. The one or more additional analytes can be citrulline, dimethylamine, arginine, orthnithine, homocysteine, and creatine. The one or more additional analytes can be amino acids or acylcarnitines. The one or more additional analytes can be measured, e.g., using mass spectrometry. The biological sample can be, or contain, blood, serum, plasma, lymph, amnionic fluid, saliva, cerebral-spinal fluid, lacrimal fluid, mucus, urine, sputum, or sweat. The biological sample that is ionized can be, or contain, compounds in addition to dimethylarginine. The subject can be on who has, or is suspected of having, a vascular disorder. The vascular disorder can a hypertensive disorder such as preeclampsia. The vascular disorder can be a cardiovascular disorder such as atherosclerosis. The vascular disorder can be diabetes, hypercholesterolemia, renal insufficiency, or hypertension.

In some embodiments, the level of ADMA and the level of SDMA can be measured.

In another aspect, the disclosure provides a method of providing a metabolic profile for a subject, the method comprising: providing a biological sample from a subject; ionizing the biological sample to generate ions; selecting ions having a mass-to-charge ratio (*m*/*z*) in an *m*/*z* range; fragmenting the selected ions to produce daughter ions; and detecting one or both of a daughter ion *m*/*z* signal at about *m*/*z* 46 and a daughter ion *m*/*z* signal at about *m*/*z* 172; and outputting a metabolic profile of the subject that includes a parameter that is a function of one or both of the daughter ion *m*/*z* signal at about *m*/*z* 46 and the daughter ion *m*/*z* signal at about *m*/*z* 172. The *m*/*z* range can be about *m*/*z* 203. The subject can be a mammal, e.g., a human. The subject can be one suspected of having a cardiovascular disorder. In some embodiments, one or more additional analytes can be measured. The one or more additional analytes can be citrulline, dimethylamine, arginine, orthnithine, homocysteine, and creatine. The one or more additional analytes can be amino acids or acylcarnitines. The one or more additional analytes can be measured, e.g., using mass spectrometry. The biological sample can be, or contain, blood, serum, plasma, lymph, amniotic fluid, saliva, cerebral-spinal fluid, lacrimal fluid, mucus, urine, sputum, or sweat. The biological sample that is ionized can be, or contain, compounds in addition to dimethylarginine. The subject can be on who has, or is suspected of having, a vascular disorder. The vascular disorder can a hypertensive disorder such as preeclampsia. The vascular disorder can be a cardiovascular disorder such as atherosclerosis. The vascular disorder can be diabetes, hypercholesterolemia, renal insufficiency, or hypertension.

In some embodiments, the level of a daughter ion *m*/*z* signal at about *m*/*z* 46 and the presence of a daughter ion *m*/*z* signal at about *m*/*z* 172 can be measured.

In some embodiments, one or more additional analytes can be measured. The one or more additional analytes can be any of those described herein. The one or more additional analytes can be measured using mass spectrometry.

In yet another aspect, the disclosure features a metabolic profile for a subject obtained by the method comprising: providing a biological sample from a subject; ionizing the biological sample to generate ions; selecting ions having a mass-to-charge ratio *(m*/*z)* in an *m*/*z* range; fragmenting the selected ions to produce daughter ions; and measuring one or both of the level of ADMA as a function of a daughter ion *m*/*z* signal at about *m*/*z* 46 and the level of SDMA as a function of a daughter ion *m*/*z* signal at about *m*/*z* 172 to obtain the metabolic profile of the subject. The *m*/*z* range can be about m/z 203. The subject can be a mammal, e.g., a human. The subject can be one suspected of having a vascular disorder. In some embodiments, one or more additional analytes can be measured. The one or more additional analytes can be any of those described herein. The one or more additional analytes can be measured using mass spectrometry.

In another aspect, the disclosure provides a method of diagnosing a vascular disorder in a subject, the method comprising: providing a metabolic profile described herein; and comparing one or both of the level of ADMA and the level of SDMA in the metabolic profile to the level of ADMA and the level of SDMA in a reference metabolic profile, wherein an elevated level of one or both of ADMA and SDMA as compared to the level in the reference metabolic profile is an indication that the subject has, or is at risk of developing, a vascular disorder.

In another aspect, the disclosure provides a method of evaluating a compound, the method comprising: contacting a cell or a subject with the compound; ionizing a sample from the cell or subject to generate ions; selecting ions having a mass-to-charge ratio (*m*/*z*) in an *m*/*z* range; fragmenting the selected ions to produce daughter ions; and measuring the level of one or both of ADMA as a function of a daughter ion *m*/*z* signal at about *m*/*z* 46 and the level of SDMA as a function of a daughter ion *m*/*z* signal at about *m*/*z* 172, wherein a change in one or both of the level of ADMA and the level of SDMA in the sample obtained from the cell or subject contacted with the compound as compared to the level in a sample from a cell or subject not contacted with the compound indicates that the compound is a compound that is a modulator of ADMA or SDMA levels. The cell can be a mammalian cell, e.g., a human cell. The cell can be an endothelial cell. The cell can be one expressing eNOS. The cell can be obtained from a subject having, or at risk of developing, a vascular disorder.

In some embodiments, one or more additional analytes can be measured. The one or more additional analytes can be any of those described herein. The one or more additional analytes can be measured using mass spectrometry.

In another aspect, the disclosure features a kit for detecting asymmetric dimethylarginine (ADMA) and symmetric dimetylarginine (SDMA), the kit comprising: one or both of ADMA and SDMA, wherein one or both ADMA and SDMA comprise at least one heavy atom isotope; and, optionally, instructions for how to detect SDMA and ADMA. The kit can contain an organic acid such as oxalic acid.

In another aspect, the disclosure features a kit for asymmetric dimethylarginine (ADMA) and symmetric dimetylarginine (SDMA), the kit comprising: one or both of ADMA and SDMA; wherein one or both ADMA and SDMA comprise at least one heavy atom isotope; an organic acid; and, optionally, instructions for how to detect SDMA and ADMA. The organic acid can be oxalic acid.

In some embodiments of any of the kits described herein, the kits can also contain computer software useful for detecting one or both of ADMA or SDMA.

In some embodiments of any of the kits described herein, the kits can also contain one or more additional internal standards useful in detecting one or more of any of the biomolecules (e.g., metabolic biomolecules) described herein.

The methods described herein have a variety of applications. For example, the methods can be used to evaluate the purity of a sample, e.g., the purity of a product of chemical synthesis or of a purification. It can be used to determine a chemical reaction, e.g., in which a dimethylarginine isomer is a reactant, intermediate or product. The reaction may occur in vitro, e.g., in a cell free system or in cells, or in vivo.

This disclosure also features a composition that includes ADMA containing at least one heavy atom isotope, and which is at least 10, 20, 60, 80, 90, 95, 97, 98, 98, 99.5% pure. The composition can have a concentration greater than about 1 pM (e.g., greater than about 1 nM). For example, the ADMA molecule has an atomic weight at least 1, 2, 3, 4, atomic units greater than naturally occurring ADMA. The composition can be substantially free of ADMA that has a mass/charge ratio of about 203. The composition can be used as an internal standard. For example, the composition can be added to a sample.

This disclosure also features a composition that includes SDMA containing at least one heavy atom isotope, and which is at least 10, 20, 60, 80, 90, 95, 97, 98, 98, 99.5% pure. The composition can have a concentration greater than about 1 pM (e.g., greater than about 1 nM). For example, the SDMA molecule has an atomic weight at least 1, 2, 3, 4, atomic units greater than naturally occurring SDMA. The composition can be substantially free of SDMA that has a mass/charge ratio of about 203. The composition can be used as an internal standard. For example, the composition can be added to a sample.

The disclosure further features a composition that includes ADMA containing at least one heavy atom isotope and SDMA containing at least one heavy atom isotope. Typically, these heavy isomers of dimethylarginine have a different mass/charge ratio from one another. The composition can be used as an internal standard. For example, the composition can be added to a sample. In one embodiment, the composition is substantially free of ADMA that has a mass/charge ratio (*m*/*z*) of about 203. In another embodiment, the composition further includes SDMA that as a mass/charge ratio of about 203.

Many mass spectrometers have mass accuracies to high resultion. For example, in the case of a singly charged ion (e.g., a singly charged ADMA or SDMA ion), this range corresponds to 0.6 *m*/*z*. In this disclosure, the singly positively charged ions of the dimethlyarginine isomers have a nominal *m*/*z* of 203. Accordingly, such ranges can be used herein. For example, the selection of ions having a mass-to-charge ratio (*m*/*z*) of about 203 can be implemented by using a channel that ranges from 202.7 to 203.3. It is understood that the methods described herein also embrace the use of multiply (e.g., doubly or triply) charged dimethylarginine ions, which multiply charged ions would effect the mass accuracy and resolution amu range accordingly. For example, for a doubly charged ion, the amu range would correspond to 0.3 *m*/*z* and so forth. Minor variations (e.g., variations in the calibration) in a mass spectrometer may result in dimethylarginine isomer parent and daughter ion m/z signals that do not coincide with the ones stated herein, but the *m*/*z* signal corresponding to those disclosed can be easily identified and used, e.g., by compensating for offset in calibration.

The materials, methods, and examples disclosed herein are illustrative only and not intended to be limiting.

Other features and advantages of the invention will be apparent from the following description, from the drawings, and from the claims.

### BRIEF DESCRIPTION OF THE DRAWINGS

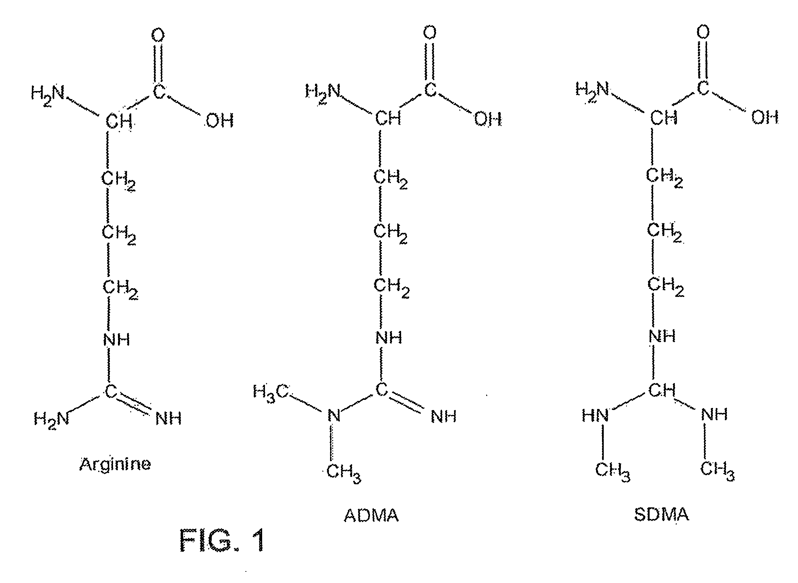

FIG. 1 is a schematic depicting the chemical structure of arginine, asymmetric dimethyl arginine (ADMA), and symmetric dimethylarginine (SDMA).

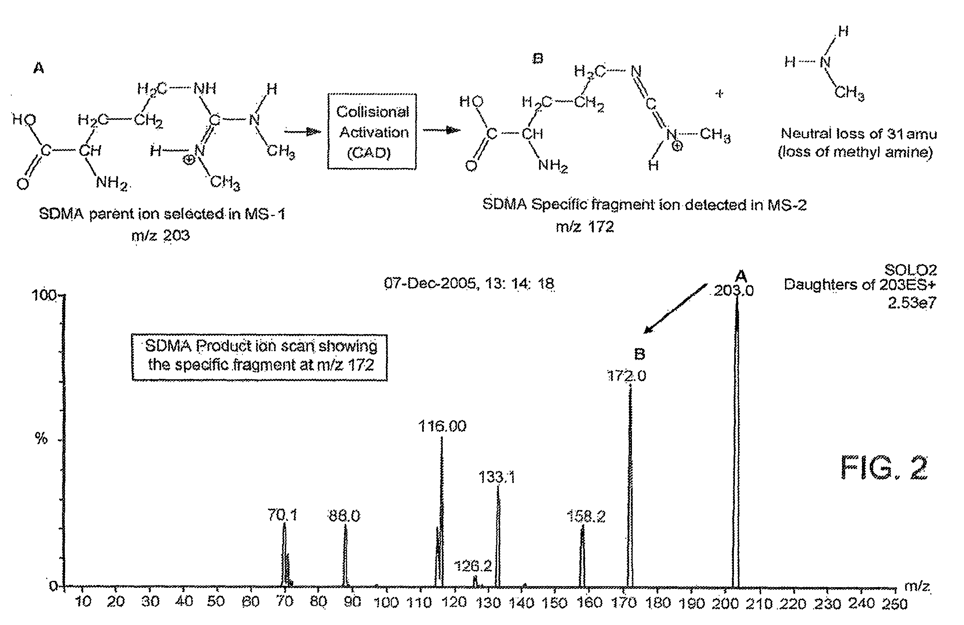

FIG. 2 is a tandem mass spectrum of SDMA and schematic depicting the fragmentation of SDMA and fragments (ionized and neutral) produced from the fragmentation. "A" indicates the parent ion SDMA having a mass-to-charge ratio (*m*/*z*) of 203. "B" indicates the SDMA specific daughter fragment ion having *m*/*z* 172. The mass spectrum depicts daughter ion *m*/*z* signals detected following fragmentation of a sample containing SDMA including the parent ion (*m*/*z* 203) and the SDMA specific fragment (*m*/*z* 172; indicated by arrow). The X-axis represents the mass-to-charge ratio (*m*/*z*) and the Y-axis represents the relative abundance (percentage) of each ion in the sample.

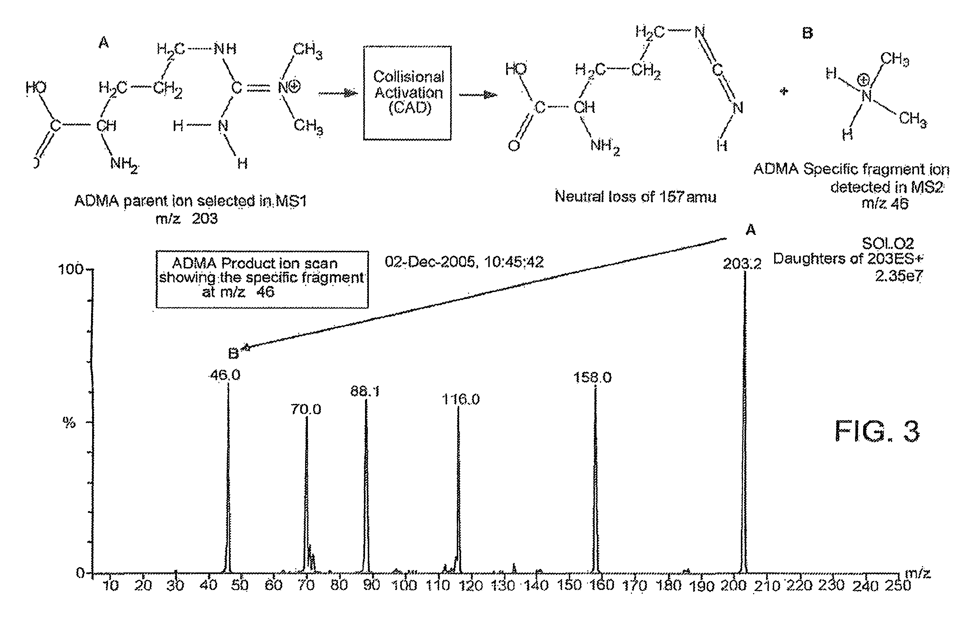

FIG. 3 is a tandem mass spectrum of ADMA and schematic depicting the fragmentation of ADMA and fragments (ionized and neutral) produced from the fragmentation. "A" indicates the parent ion ADMA having a mass-to-charge ratio (*m*/*z*) of 203. "B" indicates the ADMA specific daughter fragment ion having *m*/*z* 46. The mass spectrum depicts daughter ion *m*/*z* signals detected following fragmentation of a sample containing ADMA including the parent ion (*m*/*z* 203) and the ADMA specific fragment (*m*/*z* 46; indicated by arrow). The X-axis represents the mass-to-charge ratio (*m*/*z*) and the Y-axis represents the relative abundance (percentage) of each ion in the sample.

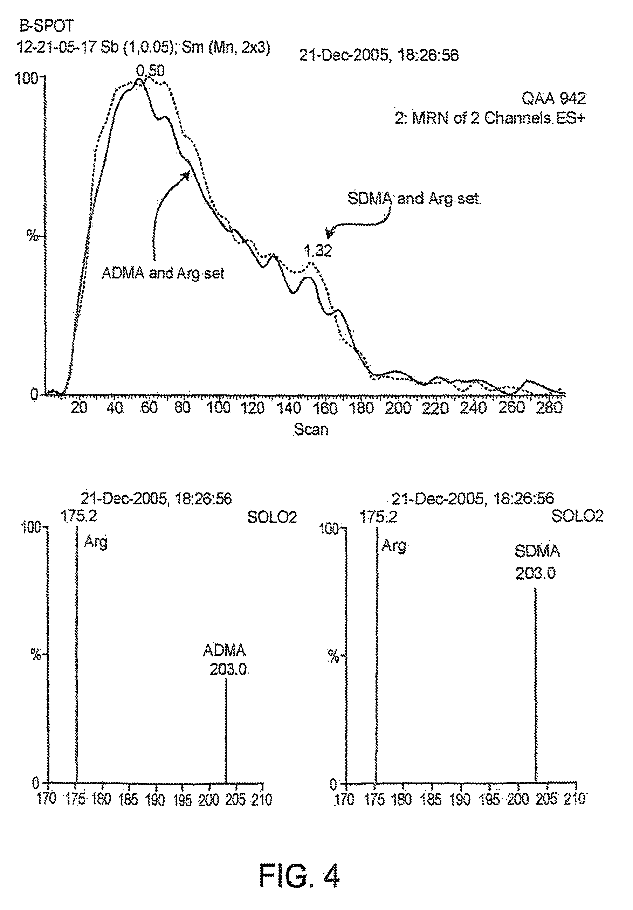

FIG. 4 is a tandem mass spectrum (experiment known as multiple reaction monitoring or MRM) of a blood sample showing the superimposed total ion current (TIC) time profiles of SDMA and ADMA (top; Multiple Reaction Monitoring) and a pair of correlate mass spectra showing the relative amount of the ADMA (left) and SDMA (right) in the sample with respect to arginine (control). The respective TIC time profiles for ADMA and SDMA are indicated by arrows (top). The X-axis of the TIC profiles is in units of number of scans and the Y-axis represents the relative abundance. For the bottom two mass spectra, the X-axes represent the mass-to-charge ratio (*m*/*z*) and the Y-axes represent the relative abundance (percentage) of each ion in the sample.

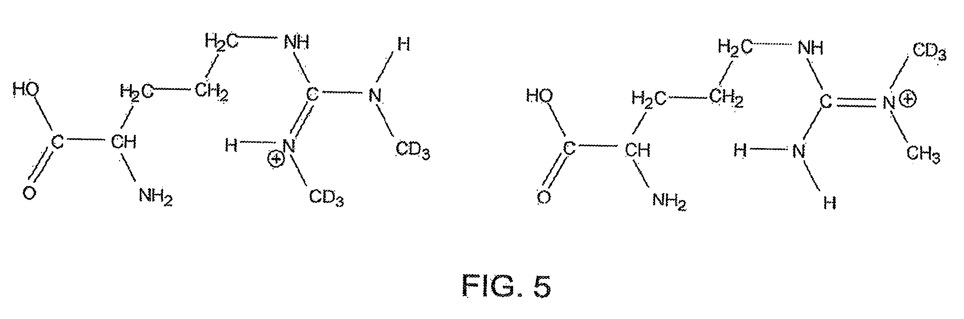

FIG. 5 is a schematic depicting the chemical structures of two internal standards useful in the methods described herein: deuterium-labeled symmetric dimethylarginine (SDMA; left) and deuterium-labeled asymmetric dimethylarginine (ADMA; right).

### DETAILED DESCRIPTION

The technology described herein relates to detecting one or more forms of molecular isomers in a manner that uniquely identifies each isomer. In one embodiment, the method includes ionizing a sample to generate ions; selecting ions having a mass-to-charge ratio (*m*/*z*) in the *m*/*z* range corresponding to the set of isomers; fragmenting the selected ions to produce daughter ions; and detecting one, two or more of the isomers by detecting at least one *m*/*z* signal for a daughter ion unique to one of the isomers. In many implementations, the method enables detecting simultaneously each isomer of the set of isomers.

The methods described herein can be used to distinguish between (and measure) dimethylarginine isomers (SDMA and/or ADMA). In applications of these methods, metabolic profiles can be obtained for a subject (e.g., a human), which profile reflects the status of at least asymmetric dimethylarginine (ADMA) and/or symmetric dimethylarginine (SDMA). The resultant metabolic profile can be used for assessing the health state of a subject (e.g., a human), such as presence or absence of a metabolic disorder or a vascular disorder (e.g., a cardiovascular disorder or a hypertensive disorder).

### Mass Spectrometry

Tandem mass spectrometry can be used to distinguish and/or measure isomers. In tandem mass spectrometry, two mass analyzers are linked in series via a collision cell. The first mass analyzer (MS-1) is used to select an ion of interest (e.g., an ion of a particular mass-to-charge ratio (*m*/*z*)). The selected ions are then transferred to a collision cell where they are fragmented by collisions with an inert gas. This process is called collisionally activated dissociation (CAD). Once the parent (sometimes referred to as precursor) ions have fragmented, the second mass analyzer (MS-2) is used to either scan and detect all of the produced daughter ions or to select and detect particular fragment ions.

In the Example 1 (below), tandem mass spectrometry was used to isolate the precursor ions of ADMA and SDMA, fragment the ions, and detect specific peaks that are indicative of the presence of these two compounds in the sample. SDMA and ADMA are isomeric molecules (see FIG. 1) and, as such, have the same molecular weight (see FIGs. 2 and 3). However, the two molecules differ by the position of their two methyl groups. As shown in FIG. 1, SDMA has a symmetrical distribution of the methyl substitution with one methyl group on each of the available guanidino nitrogen moieties, whereas ADMA contains two methyl groups on the same nitrogen of the guanidino group.

In one type of tandem mass spectrometry, known as product ion scan, parent ions (in this case SDMA *m*/*z* 203 or ADMA *m*/*z* 203) are selected in MS-1 and transferred to a collision cell where they fragment (exemplified by the schematics in FIGs. 2 and 3). The fragments produced by each parent ion are detected by scanning MS-2 resulting in a product (or daughter) ion scan of each selected parent. FIG. 2 shows a unique peak corresponding to SDMA at 172 *m*/*z,* while FIG. 3 shows a unique peak corresponding to ADMA at 46 *m*/*z.* Thus, SDMA and ADMA can be distinguished in a single sample in one analysis.

FIG. 4 shows that SDMA, ADMA and arginine can be distinguished in a single sample in one analysis using Multiple Reaction Monitoring (MRM scan). In a type of tandem mass spectrometry known as multiple reaction monitoring (MRM), a parent ion of interest is selected in MS-1, fragmented in the collision cell and a specific fragment ion resulting from the collisional activation is selected in MS-2 and finally detected. MS-1 and MS-2 are fixed to respectively select the corresponding parent and fragment ion pairs of interest for a predetermined amount of time (a few milliseconds). This specific parent ion-product ion transition can be considered as one detection channel. If additional analytes need to be detected, additional detection channels with specific mass transitions can be introduced in the experiment. The data from all selected mass transitions (channels) can be acquired sequentially to obtain the desired information. The detection and quantitation of SDMA and ADMA in a mixture can be obtained by employing the specific mass transition for each of these compounds as follows: for ADMA: MS-1 fixed to select and transmit the parent ion at *m*/*z* 203, MS-2 fixed to select and transmit the specific product ion at *m*/*z* 46 (channel 1 or MRM transition 1); and for SDMA: MS-1 fixed to select and transmit the parent ion at *m*/*z* 203, MS-2 fixed to select and transmit the specific product ion at *m*/*z* 172 (channel 2 or MRM transition 2). These two MRM transitions can be measured sequentially from the same sample for a predetermined amount of time to detect the presence and/or concentration of a mixture of these compounds in such sample. In such MRM experiments, the actual fragment ions (either *m*/*z* 46 or *m*/*z* 172) are detected. However, it is the *m*/*z* of the corresponding parent ion that is recorded in the MRM spectrum.

Due to the fact that, in this example, the SDMA and ADMA parent ions have the same *m*/*z*, each channel generates its own spectrum providing full resolution between these two isomers. The spectra shown in FIG. 4 were acquired as described above, however, mass transitions specific for arginine were added to each channel to show that when the *m*/*z* of the parent ions differs from the mass transition of the analytes, both mass transitions can be recorded in the same spectrum. These methods can also be used to distinguish between other molecular isomers, provided that, upon fragmentation, ions with different *m*/*z* ratios are produced.

The concurrent detection of arginine (e.g., as shown in FIG. 4) demonstrates that stable isotope-labeled internal standards for one or both of ADMA and SDMS can be added to a sample, by which quantitation of ADMA and SDMA can be performed. Such labeling of ADMA and SDMA with stable isotopes results in a mass shift, while retaining very similar physicochemical properties between the labeled and unlabeled compounds.

Generally, one or more internal standards can be added at known concentration to a sample to allow for quantitation of the analyte of interest (e.g., ADMA and/or SDMA). For example, for a sample analyzed using tandem mass spectrometry, the ratio of the signals produced by ADMA, SDMA and their corresponding internal standards can be used to determine the amounts of these compounds in the sample. The internal standards can also be added to distinguish naturally occurring (endogenous) molecules. The internal standards can be prepared in an extraction solution prior to mixing a sample (e.g., a blood sample) and the extraction solution. Alternatively, the internal standards can be added to the mixture at any step in the sample preparation that ensures these internal standards will not be removed from the mixture during the sample processing (e.g. after a liquid-liquid extraction or a solid phase extraction).

Internal standards, or reference analytes, for an isomer (or other molecules, e.g., biomolecules described herein) detected by a method described herein can be any modification or analog of an isomer that is detectable by mass spectrometry. A reference analyte is separately detectable from the biomolecule based on unique physical characteristics, such as a unique mass or mass-to-charge ratio. As described *supra*, a commonly used internal standard for mass spectrometry is a stable isotopically labeled form or chemical derivative of an isomer. For example, stable isotope labeled analogs can be used to quantitate SDMA and ADMA using the technique known as isotope dilution mass spectrometry where the analyte and internal standards are processed in the same sample. Internal standards can be designed such that 1) the labeling causes a shift in mass of at least 1 mass unit and 2) that none of the stable isotope labels are located in labile sites to prevent exchange. Labels can be ²H (D), ¹⁵N, ¹³C or ¹⁸O in any combination. The actual location of the labels on the molecule can vary provided the pre-requisite 2 (above) is satisfied. Moreover, the position of the labels and the potential change in the mass of the fragment ions can also be used to confirm separation of the internal standard and analytes. Examples of potential internal standards useful in the methods described herein include, but are not limited to:

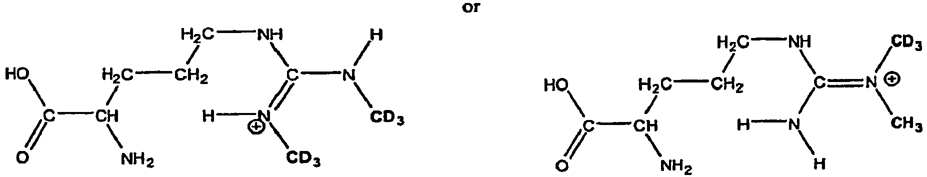

Several types of mass spectrometers are available or can be produced with various configurations, all of which can be useful in the methods described herein. In general, a mass spectrometer has the following major components: a sample inlet, an ion source, a collision cell, a mass analyzer, a detector, a vacuum system, and instrument-control system, and a data system. Difference in the sample inlet, ion source, and mass analyzer generally define the type of instrument and its capabilities. For example, an inlet can be a capillary-column liquid chromatography source or can be a direct probe or stage such as used in matrix-assisted laser desorption. Common ion sources are, for example, electrospray, including nanospray and microspray or matrix-assisted laser desorption. Common mass analyzers include quadrupole mass filters, a time-of-flight mass analysers (preferably an orthogonal acceleration time-of-flight mass analyser), ion trap mass filters, a magnetic sector analysers, or Fourier Transform Ion Cyclotron Resonance ("FTICR") mass analysers. The collision cell can be, e.g., a quadrupole rod set, a hexapole rod set, or an octopole rod set. The collision cell preferably forms a substantially gas-tight enclosure apart from an ion entrance and ion exit aperture. A collision gas such as helium, argon, nitrogen, air or methane may be introduced into the collision cell.

The specific examples described herein were performed using tandem mass spectrometers (see, e.g., Example 1).

Suitable samples for the methods described herein include any biological fluid, cell, tissue, or fraction thereof, that includes biomolecules indicative of a metabolic state. A sample can be, for example, a specimen obtained from a subject (e.g., a mammal such as a human) or can be derived from such a subject. For example, a sample can be a tissue section obtained by biopsy, or cells that are placed in or adapted to tissue culture. Exemplary samples therefore include cultured fibroblasts, cultured amniotic fluid cells, and chorionic villus sample. A sample can also be a biological fluid specimen such as urine, blood, plasma, serum, saliva, semen, sputum, cerebral spinal fluid, tears, mucus, and the like. A sample can be further fractionated, if desired, to a fraction containing particular cell types. For example, a blood sample can be fractionated into serum or into fractions containing particular types of blood cells such as red blood cells or white blood cells (leukocytes). If desired, a sample can be a combination of samples from a subject such as a combination of a tissue and fluid sample, and the like. Methods for obtaining samples that preserve the activity or integrity of molecules in the sample are well known to those skilled in the art. Such methods include the use of appropriate buffers and/or inhibitors, including nuclease, protease and phosphatase inhibitors, which preserve or minimize changes in the molecules in the sample. Such inhibitors include, for example, chelators such as ethylenediamne tetraacetic acid (EDTA), ethylene glycol bis(P-aminoethyl ether)N,N,N1,N1-tetraacetic acid (EGTA), protease inhibitors such as phenylmethylsulfonyl fluoride (PMSF), aprotinin, leupeptin, antipain and the like, and phosphatase inhibitors such as phosphate, sodium fluoride, vanadate and the like. Appropriate buffers and conditions for isolating molecules are well known to those skilled in the art and can be varied depending, for example, on the type of molecule in the sample to be characterized (see, for example, Ausubel et al. Current Protocols in Molecular Biology (Supplement 47), John Wiley & Sons, New York (1999); Harlow and Lane, Antibodies: A Laboratory Manual (Cold Spring Harbor Laboratory Press (1988); Harlow and Lane, Using Antibodies: A Laboratory Manual, Cold Spring Harbor Press (1999); Tietz Textbook of Clinical Chemistry, 3rd ed. Burtis and Ashwood, eds. W.B. Saunders, Philadelphia, (1999)). A sample also can be processed to eliminate or minimize the presence of interfering substances. For use in the methods described herein, a sample can be in a variety of physical states. For example, a sample can be a liquid or solid, can be dissolved or suspended in a liquid, can be in an emulsion or gel, and can be absorbed onto a material. As a non-limiting example, a sample can be a liquid blood sample, liquid serum sample, liquid white blood cell sample, dried blood, serum, or white cell sample, or such a sample absorbed onto a paper or polymer substrate.

Prior to performing mass spectrometry, a sample can be extracted using an extraction solution (see below). For the methods described herein, a sample (e.g., an extracted sample) does not require further chemical modification prior to analysis using tandem mass spectrometry. For example, a particular derivatization step is not requisite for specific, simultaneous detection of ADMA and SDMA. However, if the detection of other analytes in the sample requires some type of sample derivatization, this derivatization can be applied to SDMA and ADMA if appropriate or suitable. In this case, the mass of the parent ions for SDMA and ADMA will change but the specific nature of the product ions from SDMA and ADMA will not. For example, if an esterification step is required to detect and measure a group of analytes such as acylcarnitines or amino acids, SDMA and ADMA can also be esterified, which esterification would not effect the specific, simultaneous detection of ADMA and SDMA or the additional analytes. More specifically, if the analytes extracted need to be converted to butyl esters, SDMA and ADMA can also be converted to butyl esters. In this case the mass and thus the *m*/*z* for SDMA and ADMA will change from 203 to 259. However, the production of the *m*/*z* peaks at 46 and 172 specific for ADMA and SDMA, respectively, are unchanged. Therefore, derivatized SDMA and ADMA can be detected by monitoring the MS-1 and MS-2 MRM channels 259 to 46 for ADMA and 259 to 172 for SDMA. A butylation step can be used for the analysis of amino acids and acylcarnitines present in whole blood samples dried on filter paper (or other samples). Accordingly, mass spectrometry can be used to detect SDMA and ADMA from such samples at the same time as, e.g., amino acids, acylcarnitines, and other analytes.

In some applications of the methods described herein, an organic acid such as oxalic acid can be added to an extraction solution or directly to a sample. For example, the organic acid can be added to a final concentration of about 0.1% (e.g., about 0.01%, about 0.05%, about 0.15%, about 0.2%, about 0.25%, about 0.3%, about 0.4%, about 0.45%, or about 0.5%) v/v in the extracted sample. If the organic acid is added to the sample before extraction, the conditions can be minimize the loss of the organic acid. In some applications, an organic acid can be added to a solution (e.g., a cleaning or rinse solution) injected into the machine following a sample analysis.

### Exemplary Applications

The methods described herein can be used to obtain a molecular profile for a sample. The profile can include information that indicates whether a particular molecular isomer is present and typically includes information about the presence (either qualitative or quantitative) of each isomer of a particular set.

In some applications of these mass spectrometry methods, metabolic profiles for a subject (e.g., a human) can be obtained. For example, the profiles can include the level of ADMA and/or SDMA in a subject (e.g., a human patient). Other biomolecules can also be detected, quantitated, and/or evaluated, including, e.g., one or more of NMMA, ornithine, citrulline, homocycteine and creatinine, in a biological sample using tandem mass spectrometry. The resultant information (metabolic profile) can be used for assessing the health state of a subject (e.g., a human patient), such as presence or absence of a disorder, e.g., metabolic or vascular (e.g., hypertensive or cardiovascular disorder), or for evaluating risk for a disorder.

The metabolic state of a subject (e.g., a mammal such as a human) can be reflected by, e.g., the presence, amount, ratios, and modifications of amino acids within the body. Post-translational methylation is an example of an amino acid modification that can be indicative of a metabolic state. The main methylation products produced during the course of protein turnover are ADMA and SDMA. These methylated forms of arginine have roles in the normal function of an organism, and thus abnormal increases or decreases in their amounts can alter physiological functions within an organism. As an example, ADMA, as well as another arginine derivative, NMMA (monomethyl arginine), are known inhibitors for the enzyme, endothelial nitric oxide synthase (eNOS). eNDS has a role in regulating endothelial and cardiovascular functions (see, e.g., King et al. (1995) Reprod. Fertil. Dev. 7(6):1581-1584 and Jin et al. (1996) J Cardiovasc. Pharmacol. 28(3):439-446). When ADMA concentrations in an organism increase, eNDS activity is inhibited, and the physiological result is decreased endothelial function, a condition that occurs in many diseases including hypertension, pre-eclampsia, diabetes, renal insufficiency and hypercholesterolemia. SDMA does not inhibit eNOS, but has the same pathway for cell entry as ADMA and may thus affect endothelial function. As one example, several studies have shown altered SDMA levels in subjects with renal disorders (see for example Lluch P. et al. (2006) Experimental Biology and Medicine 231:70-75 and Kielstein J.T. et al. (2006) Nephrology Dialysis Transplantation 21(9):2446-2451.)

The amount and/or ratios of other amino acids can also reflect a state of endothelial function and corresponding disorders. For example, arginine is converted to citrulline and nitric oxide, which is another mediator in endothelial function. Further, ADMA is converted to citrulline and dimethylamine (DMA). The amount of arginine and/or citrulline can thus reveal a state of endothelial cell function. Various studies have shown relationships of levels of ADMA and other biomolecules and disease states. For example, Millatt et al. ((2003) Circulation 108(12):1420-1) showed data demonstrating that the rat chronic hypoxia-induced pulmonary hypertension model is associated with increased pulmonary concentrations of the NOS inhibitor ADMA. Moreover, pulmonary hypertensive rats exhibited reduced pulmonary expression and activity of the ADMA-metabolizing enzyme DDAH I. Homocysteine and creatinine are other biomolecules that play roles in vascular disease. For example, studies have shown that determining homocysteine levels can help in cardiovascular risk assessment in hypertensive populations (see, for example, Bortolotto et al. (1999) Hypertension 34(4 Pt 2):837-42). Moreover, research has shown that when nitric oxide synthase is inhibited, there is insufficient nitric oxide produced. Nitric oxide is a vasodilator; reduced nitric oxide levels have been shown to be related to hypertension. Therefore, knowledge of the amount of such biomolecules can be used as a predictor for risk of developing a hypertensive or cardiovascular disorder.

Therefore, the ability to distinguish dimethylarginine isomers -- ADMA and SDMA - either alone or together with other biomolecules (e.g., metabolic biomolecules), can be useful for assessing the health state of a subject. Hence, it is possible to, at the same time, detect other amino acids such as NMMA, arginine, ornithine, citrulline, and homocycteine, as well and other biomolecules such as creatinine, in the sample, e.g., by identifying unique peaks for such molecules in the mass spectrometry analysis. Table 1 includes a non-exhaustive list of analytes (e.g., biomolecules) that can simultaneously detected with ADMA and/or SDMA using the methods described herein.

**Table 1.**

| **ANALYTE NAME** | **ABBREVIATION** |
|---|---|
| Amino acids | |
| Alanine | Ala |
| Arginine | Arg |
| Citrulline | Cit |
| Glycine | Gly |
| Leucine | Leu |
| Methionine | Met |
| Ornithine | Orn |
| Phenylalanine | Phe |
| Tyrosine | Tyr |
| Valine | Val |

| Carnitines | |
|---|---|
| Free carnitine | C0 |
| Acetylcarnitine | C2 |
| Propionylcarnitine | C3 |
| Malonylcamitine | C3DC |
| Butyrylcarnitine | C4 |
| 3-Hydroxy-butyrytcarnitine | C4OH |
| Isovalerylcarnitine | C5 |
| Tiglylcarnitine | C5:1 |
| Glutarylcarnitine | C5DC |
| 3-Hydroxy-isovalervicarnitine | C5OH |
| Hexanoylcernitine | C6 |
| Adipylcarnitine | C6DC |
| Octanoylcernitine | C8 |
| Octenoylcarnitine | C8:1 |
| Decanoylcarnitine | C10 |
| Decenoylcarnitine | C10:1 |

A metabolic profile obtained by the methods described herein can be used in diagnosing or predicting susceptibility to a variety of metabolic or vascular (e.g., cardiovascular or hypertensive) disorders because the biochemical indicators (e.g., SDMA and/or ADMA) examined can be indicative of such disorders, whether or not physiologic or behavioral symptoms of the disorder have become apparent (e.g., one suspected of having a metabolic or vascular (e.g., cardiovascular or hypertensive disorder). A metabolic profile as described herein can be useful for monitoring the metabolism of a subject (e.g., a mammal such as a human), such as one undergoing treatment for a metabolic disorder. As a non-limiting example, the methods can be used for determining therapeutic efficacy of a particular treatment. Based on this determination, the subject can be offered additional or alternative therapeutic options. The metabolic profile can also be useful for assessing patient compliance with a particular treatment modality, such as dietary restriction. Therefore, the technology described herein is applicable to screening, diagnosis, prognosis, monitoring therapy and compliance, and any other application in which determining the presence or amount of panels of two or more biomolecules, such as two or more of ADMA, SDMA, NMMA, arginine, ornithine, citrulline, homocysteine and creatinine, is useful.

A metabolic profile generated using the methods described herein can be obtained using a variety of biological samples. Suitable samples include those described above.

In one aspect, a metabolic profile as described herein can be used to assess the presence or absence of a hypertensive disorder. Hypertensive disorders are the most common medical complications of pregnancy, and the major cause of maternal and infant disease and death worldwide. They include at least two different entities: One (pregnancy-induced hypertension, PIH) appears for the first time during pregnancy and is reversed by delivery. The other (chronic hypertension), is a preexisting condition unrelated to but coinciding with pregnancy, which may be unmasked for the first time during pregnancy and which does not resolve with delivery. Regardless of pregnant or non-pregnant state, hypertension is in many cases the result of small vessels' spasm (vasoconstriction). Therefore, the major risks to the fetus result from decreased placental perfusion leading to decreased supply of oxygen and nutrients necessary for fetal growth and well-being. Maternal risks include hypoperfusion of major organs such as kidney, liver, and brain. Hypertensive disorders include, e.g., preeclampsia, eclampsia, hypertension in pregnancy (HIP), Goldblatt hypertension, adrenal hypertension, malignant hypertension, ocular hypertension, renal hypertension, or chronic hypertension. Hypertensive disorders can result in brain or renal edema and hemorrhage and stroke.

In further aspects, a metabolic profile as described herein can be used to assess the presence or absence of cardiovascular disorders, such as hyperhomocysteinemia, diabetes mellitus, hypercholesterolemia, hyperglycemia and insulin resistance, coronary heart disease, cerebrovascular disease, atherosclerosis and peripheral vascular disease. A panel of biomolecules for detection using the methods described herein can be selected based on the particular health condition to be detected. Health conditions associated with ADMA, SDMA, NNMA, arginine, citrulline, and the like, are known to those skilled in the art. As an example, health conditions associated with ADMA and other biomolecules are described in Lin and Lin (2004) Acta Cardiol Sin 2:201-211. In some cases, a metabolic profile can be generated from a subject already identified as having or at risk of developing a metabolic or vascular disorder such as a cardiovascular or hypertensive disorder, e.g., to monitor the status of the subject's disorder, the subject's response to a particular treatment modality, or a subject's compliance with a treatment regimen.

Subjects of all ages can be affected by metabolic or vascular disorders diagnosed using a metabolic profile described herein. Therefore, a sample used in a method described herein can be obtained from a subject (e.g., a human) of any age, including a neonate, newborn, baby, child, and adult, such as a pregnant female. The methods can also be used for individuals at risk of developing a metabolic or vascular disorder. Such individuals include those who have (i) a family history of (a genetic predisposition for) such disorders or (ii) one or more risk factors for developing such disorders. Exemplary risk factors for cardiovascular or hypertensive disorders include: exposure to prolonged stress (emotional or physical), smoking, a diet high in fat or cholesterol, a sedentary lifestyle, or one or more medications that raise blood pressure.

The methods described herein involve determining the presence or amount of at least two biomolecules (e.g., two or more isomers), e.g., simultaneously, where the presence or amount of each biomolecule correlates the presence or absence of a metabolic, cardiovascular, or hypertensive disorder. The methods described herein can be used quantitatively, if desired, to allow comparison of test sample results with known or a pre-determined standard amount of a particular analyte(s) (e.g., by using an internal standard as described above). The methods can also be used qualitatively when a test sample is compared with a reference sample, which can be either a normal reference or metabolic disorder reference. In this format, the relative amount of biomolecules can be indicative of a metabolic disorder. A reference sample, for example, can be from a subject having, not suspected of having, or not at risk of developing a disorder such as a metabolic or vascular disorder (e.g., cardiovascular or hypertensive disorder).

Generally, a cut-off value for a given biomolecule can vary and would be known in the art for commonly tested analytes and enzymes. Routine, obvious adaptations of methods known in the art can be used to establish cut-off values for uncommonly tested analytes. A cut-off value is typically a biomolecule amount, or ratio with another biomolecule, above or below which is considered indicative of a metabolic disorder or cause for retest. Thus, in accordance with the technology described herein a reference level of at least one biomolecule in a particular sample type is identified as a cut-off value, above which there is a significant correlation between the presence of the at least one biomolecule and presence (or absence) of a metabolic disorder. It is understood that biomolecule panels can be interpreted as a whole, in parts or on an analyte-by-analyte basis.

Those of skill in the art will recognize that some cut-off values are not absolute in that clinical correlations are still significant over a range of values on either side of the cutoff; however, it is possible to select an optimal cut-off value (e.g. varying H-scores, and the like) of biomolecule for a particular sample types. Cut-off values determined for use in the methods described herein generally are compared with published ranges but can be individualized to the methodology used and patient population. It is understood that improvements in optimal cut-off values could be determined depending on the sophistication of statistical methods used and on the number and source of samples used to determine reference level values for the different biomolecules and sample types. Therefore, established cut-off values can be adjusted up or down, on the basis of periodic re-evaluations or changes in methodology or population distribution. In addition, instrument-specific cut-off values can be used, if desired, for example such as when inter-instrument performance comparability is <10%.

The reference level can be determined by a variety of methods, provided that the resulting reference level accurately provides an amount of each biomolecule above which exists a first group of subjects (e.g., humans) having a different probability of metabolic disorder than that of a second group of subjects having metabolic analyte or enzyme activity amount below the reference level. The reference level can be determined by comparison of biomolecule amount in, e.g., populations of subjects (e.g., patients) having the same metabolic disorder. This can be accomplished, for example, by histogram analysis, in which an entire cohort of patients are graphically presented, wherein a first axis represents the amount of biomolecule and a second axis represents the number of subjects in the cohort whose sample contain one or more biomolecules at a given amount. Two or more separate groups of subjects can be determined by identification of subsets populations of the cohort which have the same or similar levels of biomolecules. Determination of the reference level can then be made based on an amount which best distinguishes these separate groups. The reference level can be a single number, equally applicable to every subject, or the reference level can vary, according to specific subpopulations of subjects. For example, older subjects can have a different reference level than younger subjects for the same metabolic disorder. In addition, a subject with more advanced disease (e.g., a more advanced form of a cardiovascular disorder) can have a different reference value than one with a milder form of the disease.

### Methods of Identifying Compounds that Modulate ADMA or SDMA levels

Also provided herein are methods of identifying compounds that modulate (e.g., decrease) the levels of ADMA and/or SDMA in a cell. Since disregulagted levels of these two metabolites are associated with increased risk of certain disorders (e.g., cardiovascular and hypertensive disorders), compounds so identified could be useful in treating cardiovascular and hypertensive disorders. Cells that can be contacted with the candidate compound can be of any species such that the cells produce ADMA or SDMA (either synthetically or naturally). The cells can be primary cells or cell lines and can be of any histological type, e.g., without limitation, epithelial cells, fibroblasts, lymphoid cells, macrophages/monocytes, granulocytes, keratinocytes, neuronal cells, or muscle cells. The cells can be cultured in tissue culture dishes. Often it is preferable to grow the cells in multiwell assay plates (e.g., 96 well or 384 well assay plates) such that multiple candidate compounds can be evaluated at one time. The candidate compound (optionally at various concentrations ranging, e.g., from 0.001 nM to 10 mM) can be added to a solution (e.g., culture medium) containing the cells or, where the compound is a protein, the cells can recombinantly express it. Following incubation of cells expressing ADMA and/or SDMA, the presence or level of ADMA and/or SDMA can be determined using the mass spectrometry methods described herein. Prior to detection, the cells can be lysed under conditions that allow for a sample to be prepared which is compatible with tandem mass spectroscopy. Often a control compound can be added to a set of cells as either a positive or negative control. For example, a compound known to increase or decrease the amount ADMA or SDMA can be added to a set of cells (e.g., a compound that inhibits protein arginine methyltransferase; see, e.g., Leiper et al. (2005) Eur. J Clin. Pharma. 62(Suppl):33-35). A compound known not to modulate the level of ADMA or SDMA can also be added to a set of cells.

The compounds identified in any of the methods described herein include various chemical classes. Compounds can be biomolecules including, but not limited to, peptides, polypeptides, peptidomimetics (e.g., peptoids), amino acids, amino acid analogs, saccharides, fatty acids, steroids, purines, pyrimidines, derivatives or structural analogues thereof, polynucleotides, and polynucleotide analogs. Compounds can be both small or large molecule compounds.

Identification of test compounds through the use of the various libraries described herein permits subsequent modification of the test compound "hit" or "lead" to optimize the capacity of the "hit" or "lead" to modulate the levels of ADMA and/or SDMA in a cell.

It is understood that modifications that do not substantially affect the activity of the various embodiments of this invention are also included within the definition of the invention provided herein. Accordingly, the following example is intended to illustrate, not to limit, the present invention.

### Example 1

This example describes an experiment in which ADMA and SDMA present in a single sample were distinguished using mass spectrometry. The sample was prepared by spiking ADMA and SDMA into whole blood at approximately equimolar concentrations. Arginine was also spiked into the same aliquot of blood to serve as a relative intensity reference. The blood was then dispensed onto filter paper and allowed to dry at room temperature for 24 hours. Small punches (1/8" each) were excised from the dried blood sample and dispensed into wells of a microtiter plate. The blood spot punches thus produced were then extracted in the microtiter plate with an aqueous methanol solution (containing 80% methanol, 20%water and 0.1 % acetic acid) for approximately 30 minutes. The extract was injected directly, with the aid of an autosampler, into the mass spectrometer with out further chemical modification and without any upfront chromatographic separation (e.g., preliminary HPLC). The experiments were performed using either a Sciex API 2000 triple quadrupole or a Waters Quattro Micro triple quadrupole. Both mass spectrometers were equipped with electrospray ions sources

The tandem mass spectrometry analysis of the sample consisted of two sets of multiple reaction monitoring (MRM) channels. In one set, the following mass transitions were monitored: *m*/*z* 175 to *m*/*z* 70 and *m*/*z* 203 to *m*/*z* 46 (see FIGs. 2 and 3). The first mass transition corresponds to a specific fragmentation for arginine and the second to a specific fragmentation for ADMA. In the second set of MRM channels, arginine and SDMA were monitored by the following mass transitions: *m*/*z* 175 to *m*/*z* 70 and *m*/*z* 203 to *m*/*z* 172 for arginine and SDMA, respectively. The transition for arginine corresponds to the neutral loss of 105 atomic mass units (amu) and represents the nominal loss of the carboxylic and guanidino groups from the protonated ion. The specific transition for ADMA represents the formation of the dimethyl ammonium ion from protonated ADMA and the specific transition for SDMA represents the neutral loss of 31 amu which is equivalent to the neutral loss of the methyl amine.

Even without absolute quantitation, examination of the ADMA and SDMA intensities relative to the arginine intensities differentiates these two isomeric ions. In this example it can be seen that the intensity of SDMA relative to that of arginine is higher than that of ADMA against arginine. This trend indicates that these two isomers do exhibit differential sensitivity in the tandem mass spectrometry experiment. The measurements of arginine, SDMA and ADMA can be affected by residual (carry over) analyte molecules (e.g., ADMA or SDMA) remaining in the mass spectrometer. Addition of oxalic acid to the sample preparation significantly reduced the observed carry over effects. FIG. 4 shows an example of the MRM spectra from the example described above.

## Claims

1. A method of distinguishing dimethylarginine isomers, the method comprising:
ionizing a sample to generate ions;
selecting ions having a mass-to-charge ratio (m/z) in an m/z range;
fragmenting the selected ions to produce daughter ions; and
detecting one or both of asymmetric dimethylarginine (ADMA) and symmetric dimethylarginine (SDMA) in the sample by detecting one or both of a daughter ion m/z signal unique to ADMA and a daughter ion m/z signal unique to SDMA, wherein the sample is not subjected to a chromatographic separation prior to ionizing the sample.

2. The method of claim 1, wherein detecting one or both of a daughter ion m/z signal unique to ADMA and a daughter ion m/z signal unique to SDMA comprises detecting one or both of the presence of a daughter ion m/z signal at about m/z 46 and the presence of a daughter ion m/z signal at about m/z 172, wherein a daughter ion m/z signal at about m/z 46 indicates that the sample comprises asymmetric dimethylarginine (ADMA) and a daughter ion m/z signal at about m/z 172 indicates that the sample comprises symmetric dimethylarginine (SDMA).

3. The method of claim 1 or 2, wherein the m/z range is about m/z 203.

4. The method of any one of claims 1-3, wherein the sample comprises one or more internal standards and wherein at least one internal standard is a dimethylarginine isomer containing at least one heavy atom.

5. The method of claim 4, wherein the method comprises:
selecting ions having an m/z range corresponding to the range being suitable to isolate dimethylarginine isomers and selecting ions in one or more additional m/z ranges, the ranges being suitable to isolate the one or more internal standards; and
detecting the one or more internal standards.

6. The method of claim 5, wherein sample comprises a first internal standard that is an SDMA isomer having at least one heavy atom, and a second internal standard that is an ADMA isomer having at least one heavy atom and wherein the SDMA and ADMA isomers have different atomic weights.

7. The method of claim 5, wherein sample comprises a first internal standard that is an SDMA isomer having at least one heavy atom, and a second internal standard that is an ADMA isomer having at least one heavy atom and wherein the SDMA and ADMA isomers have the same atomic weights.

8. The method of any of claims 1-7, further comprising measuring the level of one or both of SDMA and ADMA in the sample.

9. The method of any of claims 1-8, wherein the sample that is ionized is a biological sample.

10. The method of any of claims 1-9, wherein one or more additional analytes are detected.

11. The method of claim 10, wherein the one or more additional analytes are metabolic biomolecules.

12. The method of claim 10 or 11, wherein the one or more additional analytes are selected from the group consisting of amino acids and acylcarnitines.

13. The method of claim 10 or 11, wherein the one or more additional analytes are selected from the group consisting of: citrulline, dimethylamine, arginine, orthnithine, homocysteine, and creatine.

14. The method of any of claims 1-13, wherein the sample is not chemically modified prior to ionizing the sample.

15. The method of any of claims 1-14, wherein the level of a signal at about m/z 46 and the level of a signal at about m/z 172 are measured.

16. The method of any of claims 1-15, wherein the sample comprises oxalic acid.

17. A method of diagnosing a disorder **characterized by** altered ADMA or SDMA levels, the method comprising:
providing a biological sample from a subject;
ionizing the biological sample to generate ions;
selecting ions having a mass-to-charge ratio (m/z) in an m/z range;
fragmenting the selected ions to produce daughter ions; and
measuring the level of ADMA as a function of a daughter ion m/z signal at about m/z 46 and the level of SDMA as a function of a daughter ion m/z signal at about m/z 172,
wherein an altered level of one or both of ADMA and SDMA in the biological sample relative to the level in a reference sample is an indication that the subject has, or is at risk of developing, a disorder **characterized by** altered ADMA or SDMA levels,
wherein the disorder **characterized by** altered ADMA or SDMA levels is preeclampsia, atherosclerosis, diabetes, hypercholesterolemia, renal insufficiency, or hypertension, and
wherein the biological sample is not subjected to a chromatographic separation prior to ionizing the biological sample.

18. The method of claim 17, wherein the disorder **characterized by** altered ADMA or SDMA levels is preeclampsia.

19. The method of claim 17 or 18, further comprising adding oxalic acid to the biological sample.

20. A method of evaluating a subject's response to a therapeutic agent, the method comprising:
providing a biological sample from a subject treated with a therapeutic agent;
ionizing the biological sample to generate ions;
selecting ions having a mass-to-charge ratio (m/z) in an m/z range;
fragmenting the selected ions to produce daughter ions; and
measuring one or both of the level of ADMA as a function of a daughter ion m/z signal at about m/z 46 and the level of SDMA as a function of a daughter ion m/z signal at about m/z 172,
wherein the biological sample is not subjected to a chromatographic separation prior to ionizing the biological sample, and
wherein the same level or an elevated level of one or both of ADMA and SDMA in the biological sample relative the level in a biological sample obtained from the subject prior to treatment is an indication that the subject is not responding to the treatment and a reduced level of one or both of ADMA and SDMA in the biological sample relative to the biological sample obtained from the subject prior to treatment is an indication that the subject is responding to the treatment.

21. The method of claim 20, further comprising adding oxalic acid to the biological sample.

22. The method of any one of claims 17 to 21, wherein the subject is a human.

23. A kit for detecting asymmetric dimethylarginine (ADMA) and symmetric dimethylarginine (SDMA), the kit comprising one or both of ADMA and SDMA, wherein one or both ADMA and SDMA comprise at least one heavy atom isotope, and oxalic acid.

## Patentansprüche

1. Verfahren zum Unterscheiden von Dimethylargininisomeren, wobei das Verfahren Folgendes umfasst:
das Ionisieren einer Probe, um Ionen zu bilden;
das Auswählen von Ionen, die ein Masse-zu-Ladung-Verhältnis (m/z) in einem m/z-Bereich aufweisen;
das Fragmentieren der ausgewählten Ionen, um Tochterionen herzustellen; und das Erfassen eines oder beider von asymmetrischem Dimethylarginin (ADMA) und symmetrischem Dimethylarginin (SMDA) in der Probe durch Erfassen eines oder beider von einem Tochterionen-m/z-Signal, das für ADMA spezifisch ist, und eines Tochterionen-m/z-Signals, das für SDMA spezifisch ist, wobei die Probe vor dem Ionisieren der Probe keiner chromatographischen Trennung unterworfen wird.

2. Verfahren nach Anspruch 1, wobei das Erfassen eines oder beider von einem Tochterionen-m/z-Signal, das für ADMA spezifisch ist, und einem Tochterionen-m/z-Signal, das für SDMA. spezifisch ist, das Erfassen von einem oder beiden des Vorliegens eines Tochterionen-m/z-Signals bei etwa m/z 46 und des Vorliegens eines Tochterionen-m/z-Signals bei etwa m/z 172 umfasst, wobei ein Tochterionen-m/z-Signal bei etwa m/z 46 anzeigt, dass die Probe asymmetrisches Dimethylarginin (ADMA) umfasst und ein Tochterionen-m/z-Signal bei etwa m/z 172 anzeigt, dass die Probe symmetrisches Dimethylarginin (SMDA) umfasst.

3. Verfahren nach Anspruch 1 oder 2, wobei der m/z-Bereich etwa m/z 203 beträgt.

4. Verfahren nach einem der Ansprüche 1-3, wobei die Probe einen oder mehrere interne Standards umfasst und wobei mindestens ein interner Standard ein Dimethylargininisomer ist, das mindestens ein Schweratom enthält.

5. Verfahren nach Anspruch 4, wobei das Verfahren Folgendes umfasst:
das Auswählen von Ionen, die einen m/z-Bereich aufweisen, der dem Bereich entspricht, der zum Isolieren von Dimethylargininisomeren geeignet ist, und das Auswählen von Ionen in einem oder mehreren zusätzlichen m/z-Sereichen, wobei die Bereiche geeignet sind, den einen oder die mehreren internen Standards zu isolieren; und
das Erfassen des einen oder mehrerer interner Standards.

6. Verfahren nach Anspruch 5, wobei die Probe einen ersten internen Standard, der ein SDMA-Isomer ist, das mindestens ein Schweratom aufweist, und einen zweiten internen Standard, der ein ADMA-Isomer ist, das mindestens ein Schweratom aufweist, umfasst, und wobei die SDMA- und ADMA-Isomere verschiedene Atomgewichte aufweisen.

7. Verfahren nach Anspruch 5, wobei die Probe einen ersten internen Standard, der ein SDMA-Isomer ist, das mindestens ein Schweratom aufweist, und einen zweiten internen Standard, der ein ADMA-Isomer ist, das mindestens ein Schweratom aufweist, umfasst, und wobei die SDMA- und ADMA-Isomere dieselben Atomgewichte aufweisen.

8. Verfahren nach einem der Ansprüche 1-7, des Weiteren das Messen des Spiegels eines oder beider von SDMA und ADMA in der Probe umfassend.

9. Verfahren nach einem der Ansprüche 1 - 8, wobei die Probe, die ionisiert wird, eine biologische Probe ist.

10. Verfahren nach einem der Ansprüche 1 - 9, wobei ein oder mehrere zusätzliche Analyte erfasst wenden.

11. Verfahren nach Anspruch 10, wobei der eine oder die mehreren zusätzlichen Analyte metabolische Biomoleküle sind.

12. Verfahren nach Anspruch 10 oder 11, wobei der eine oder die mehreren zusätzlichen Analyse aus der Gruppe ausgewählt ist/sind bestehend aus Aminosäuren und Acylcarnitinen.

13. Verfahren nach Anspruch 10 oder 11, wobei der eine oder die mehreren zusätzlichen Analyte aus der Gruppe ausgewählt ist/sind bestehend aus: Citrullin, Dimethylamin, Arginin, Ornithin, Homocystein und Creatin.

14. Verfahren nach einem der Ansprüche 1 - 13, wobei die Probe vor dem Ionisieren der Probe nicht chemisch modifiziert wird.

15. Verfahren nach einem der Anspruche 1 - 14, wobei das Niveau eines Signals bei etwa m/z 46 und das Niveau eines Signals bei etwa m/z 172 gemessen werden.

16. Verfahren nach einem der Ansprüche 1-15, wobei die Probe Oxalsäure umfasst.

17. Verfahren zum Diagnostizieren einer Gesundheitsstörung, **gekennzeichnet durch** geänderte ADMA-oder SDMA-Spiegel, wobei das Verfahren Folgendes umfasst:
das Bereitstellen einer biologischen Probe von einem Subjekt;
das Ionisieren der biologischen Probe, um Ionen zu bilden;
das Auswählen von Ionen, die ein Masse-zu-I,adung-Verhältuis (m/z) in einem m/z-Bereich aufweisen;
das Fragmentieren der ausgewählten Ionen, um Tochterionen herzustellen; und das Messen des ADMA-Spiegels in Abhängigkeit von einem Tochterionen-m/z-Signal bei etwa nr/z 46 und des SDMA-Spiegels in Abhängigkeit von einem Tochterionen-m/z-Signal bei etwa m/z 172,
wobei ein geänderter Spiegel von einem oder beiden von ADMA und SDMA in der biologischen Probe im Vergleich mit dem Spiegel in einer Bezugsprobe einen Hinweis liefert, dass das Subjekt an einer Gesundheitsstörung leidet oder dem Risiko des Entwickeln derselben ausgesetzt ist, die **durch** geänderte ADMA- oder SDMA-Spiegel gekennzeichnet ist,
wobei die Gesundheitsstörung, die **durch** geänderte ADMA- oder SDMA-Spiegel gekennzeichnet ist, Präeklampsie, Atherosklerose, Diabetes, Hyperchotcsterinämie, Nierenllsuffizienz oder hoher Blutdruck ist und
wobei die biologische Probe vor dem Ionisieren der biologischen Probe keiner chromatographischen Trennung unterworfen wird.

18. Verfahren nach Anspruch 17, wobei die Gesundheitsstörung, die durch geänderte ADMA- oder SDMA-Spiegel gekennzeichnet ist, Präeklampsie ist.

19. Verfahren nach Anspruch 17 oder 18, des Weiteren das Hinzugeben von Oxalsäure zu der biologischen Probe umfassen.

20. Verfahren zum Beurteilen der Reaktion eines Patienten auf ein therapeutisches Mittel, wobei das Verfahren Folgendes umfasst,:
das Bereitstellen einer biologischen Probe von einem Subjekt, das mit einem therapeutischen Mittel behandelt wird;
das Ionisieren der biologischen Probe, um Ionen zu bilden;
das Auswählen von Ionen, die ein Masse-zu-Ladung-Verhältnis (m/z) in einem m/z-Bereich aufweisen;
das Fragmentieren der ausgewählten Ionen, um Tochterionen herzustellen; und
das Messen eines oder beider des ADMA-Spiegels in Abhängigkeit von einem Tochterionen-m/z-Signal bei etwa m/z 46 und des SDMA-Spiegels in Abhängigkeit von einem Tochterionen-ni7z-Signal bei etwa m/z 172,
wobei die biologische Probe vor dem Ionisieren der biologischen Probe keiner chromatographischen Trennung unterworfen wird, und
wobei derselbe Spiegel oder ein erhöhter Spiegel von einem oder beiden von ADMA und SDMA in der biologischen Probe im Vergleich mit dem Spiegel in einer biologischen Probe, die von dem Subjekt vor der Behandlung erhalten worden ist, einen Hinweis liefert, dass das Subjekt nicht auf die Behandlung anspricht, und ein reduzierter Spiegel von einem oder beiden von ADMA und SDMA in der biologischen Probe im Vergleich mit einer biologischen Probe, die von dem Subjekt vor der Behandlung erhalten worden ist, einen Hinweis liefert, dass das Subjekt auf die Behandlung anspricht.

21. Verfahren nach Anspruch 20, des Weiteren das Einzugeben von Oxalsäure zu der biologischen Probe umfassend.

22. Verfahren nach einem der Ansprüche 17 bis 21, wobei das Subjekt ein Mensch ist.

23. Kit zum Erfassen von asymmetrischem Dimethylarginin (ADA) und symmetrischem Dimethylarginin (SDMA), wobei das Kit eines oder beide von ADMA und SDMA, wobei eines oder beide von ADMA und SDMA mindestens ein Schweratomsisotop umfasst/umfassen, und Oxalsäure umfasst.

## Revendications

1. Procédé pour la distinction d'isomères de diméthylarginine, le procédé comprenant:
l'ionisation d'un échantillon pour générer des ions;
la sélection d'ions possédant un rapport de masse sur charge (m/z) dans un domaine de m/z;
la fragmentation des ions sélectionnées pour produire des ions de filiation; et
la détection d'une ou des deux parmi une diméthylarginine asymétrique (ADMA) et une diméthylarginine symétrique (SDMA) dans l'échantillon par la détection d'un au des deux parmi un signal m/z d'ions de filiation unique pour ADMA et un signal m/z d'ions de filiation unique pour SDMA, dans lequel l'échantillon n'est pas soumis à une séparation chromatographique avant l'ionisation de l'échantillon.

2. Procédé selon la revendication 1, dans lequel la détection d'un ou des deux parmi un signal m/z d'ions de filiation unique pour ADMA et un signal m/z d'ions de filiation unique pour SDMA comprend la détection d'une ou des deux parmi la présence d'un signal m/z d'ions de filiation à environ m/z 46 et la présence d'un signal m/z d'ions de filiation à environ m/z 172, dans lequel un signal m/z d'ions de filiation à environ m/z 46 indique que l'échantillon comprend une diméthylarginine asymétrique (ADMA) et un signal m/z d'ions de filiation à environ m/z 172 indique que l'échantillon comprend une diméthylarginine symétrique (SDMA).

3. Procédé selon la revendication 1 ou 2, dans lequel le domaine de m/z est environ m/z 203.

4. Procédé selon l'une quelconque des revendications 1-3, dans lequel l'échantillon comprend une ou plusieurs références internes et dans lequel au moins une référence interne est un isomérie de diméthylarginine contenant au moins un atome lourd.

5. Procédé selon la revendication 4, où le procédé comprend:
la sélection d'ions ayant un domaine de m/z correspondant au domaine qui est approprié pour isoler des isomères de diméthylarginine et la sélection d'ions dans un ou plusieurs domaines de m/z supplémentaires, les domaines étant appropriés pour isoler les une ou plusieurs références internes; et
la détection des une ou plusieurs références internes.

6. Procédé selon la revendication 5, dans lequel l'échantillon comprend une première référence interne qui est un isomère de SDMA possédant au moins un atome lourd et une deuxième référence interne qui est un isomère d'ADMA possédant au moins un atome lourd et dans lequel les isomères de SDMA et d'ADMA présentent des poids atomiques différents.

7. Procédé selon la revendication 5, dans lequel l'échantillon comprend une première référence interne qui est un isomère de SDMA possédant au moins un atome lourd et une deuxième référence interne qui est un isomère d'ADMA possédant au moins un atome lourd et dans lequel les isomères de SDMA et d'ADMA présentent des poids atomiques identiques.

8. Procédé selon l'une quelconque des revendications 1-7, comprenant en outre la mesure du niveau d'une ou des deux parmi SDMA et ADMA dans l'échantillon.

9. Procédé selon l'une quelconque des revendications 1-8, dans lequel l'échantillon qui est ionisé est un échantillon biologique.

10. Procédé selon l'une quelconque des revendications 1-9, dans lequel un ou plusieurs analytes supplémentaires sont détectés.

11. Procédé selon la revendication 10, dans lequel les un ou plusieurs analytes supplémentaires sont des biomolécules métaboliques.

12. Procédé selon la revendication 10 ou 11, dans lequel les un ou plusieurs analytes supplémentaires sont choisis dans le groupe constitué d'acides aminés et d'acylcarnitines,

13. Procédé selon la revendication 10 ou 11, dans lequel les un ou plusieurs analytes supplémentaires sont choisis dans le groupe constitué de: citrulline, diméthylamine, arginine, orthnithine, homocystéine et créatine.

14. Procédé selon l'une quelconque des revendications 1-13, dans lequel l'échantillon n'est pas chimiquement modifié avant l'ionisation de l'échantillon.

15. Procédé selon l'une quelconque des revendications 1-14, dans lequel le niveau d'un signal à environ m/z 46 et le niveau d'un signal à environ m/z 172 sont mesurés.

16. Procédé selon l'une quelconque des revendications 1-15, dans lequel échantillon comprend de l'acide oxalique.

17. Procédé pour le diagnostic d'un trouble **caractérisé par** des niveaux d'ADMA ou de SDMA modifiés, le procédé comprenant:
la fourniture d'un échantillon biologique provenant d'un sujet;
l'ionisation de l'échantillon biologique pour générer des ions;
la sélection d'ions possédant un rapport masse sur charge (m/z) dans une domaine de m/z;
la fragmentation des ions sélectionnés pour produire des ions de filiation; et
la mesure du niveau d'ADMA en fonction d'un signal m/z d'ions de filiation à environ m/z 46 et du niveau de SDMA en fonction d'un signal m/z d'ions de filiation à environ m/z 172,
dans lequel un niveau modifié d'une ou des deux parmi ADMA et SDMA dans l'échantillon biologique relativement au niveau dans un échantillon de référence est une indication que le sujet présente, ou est susceptible de développer, un trouble **caractérisé par** des niveaux d'ADMA ou de SDMA modifiés,
dans lequel le trouble **caractérisé par** des niveaux d'ADMA ou de SDMA modifiés est une pré-éclampsie, une athérosclérose, un diabète, une hypercholestérolémie, une insuffisance rénale ou une hypertension, et
dans lequel l'échantillon biologique n'est pas soumis à une séparation chromatographique avant l'ionisation de l'échantillon biologique.

18. Procédé selon la revendication 17, dans lequel le trouble **caractérisé par** des niveaux d'ADMA ou de SDMA modifiés est une pré-éclampsie.

19. Procédé selon la revendication 17 ou 18, comprenant en outre l'ajout d'acide oxalique à l'échantillon biologique.

20. Procédé pour l'évaluation d'une réaction d'un sujet à un agent thérapeutique, le procédé comprenant:
la fourniture d'un échantillon biologique provenant d'un sujet traité avec un agent thérapeutique;
l'ionisation de l'échantillon biologique pour générer des iom-e
la sélection d'ions possédant un rapport de masse sur charge (m/z) dans un domaine de m/z;
la fragmentation des ions sélectionnés pour produire des ions de filiation; et
la mesure d'un ou des deux parmi le niveau d'ADMA en fonction d'un signal m/z d'ions de filiation à environ m/z 46 et le niveau de SDMA en fonction d'un signal m/z d'ions de filiation à environ m/z 172,
dans lequel l'échantillon biologique n'est pas soumis à une séparation chromatographique avant l'ionisation de l'échantillon biologique, et
dans lequel le même niveau ou un niveau élevé d'une ou des deux parmi ADMA, et SDIVIA dans l'échantillon biologique relativement au niveau dans un échantillon biologique obtenu à partir du sujet avant le traitement est une indication que le sujet ne réagit pas positivement au traitement et un niveau réduit d'une ou des deux parmi ADMA et SDMA dans l'échantillon biologique relativement à l'échantillon biologique obtenu à partir du sujet avant le traitement est une indication que le sujet réagit positivement au traitement.

21. Procédé selon la revendication 20, comprenant en outre l'ajout d'acide oxalique à l'échantillon biologique.

22. Procédé selon l'une quelconque des revendications 17 à 21, dans lequel le sujet est un humain.

23. Kit pour la détection de diméthylarginine asymétrique (ADMA) et de diméthylarginine symétrique (SDMA), le kit comprenant une ou les deux parmi ADMA et SDMA, dans lequel une ou les deux parmi ADMA et SDMA comprennent au moins un isotope d'atome lourd, et de l'acide oxalique.
